Europäisches Patentamt

European Patent Office

Office européen des brevets

(11) Publication number: **0 093 493**
**A1**

# (12) EUROPEAN PATENT APPLICATION

(21) Application number: 83301436.8

(22) Date of filing: 15.03.83

(51) Int. Cl.³: **A 61 F 9/00**
**A 61 J 1/00, B 65 D 47/18**

(30) Priority: 29.04.82 US 372967

(43) Date of publication of application:
09.11.83 Bulletin 83/45

(84) Designated Contracting States:
AT BE CH DE FR GB IT LI LU NL SE

(71) Applicant: St. Luke's Hospital of Cleveland, Ohio
11311 Shaker Boulevard
Cleveland Ohio 44104(US)

(72) Inventor: Dougherty, Delford O.
13610 Shaker Boulevard
Cleveland Ohio 44120(US)

(74) Representative: Abbie, Andrew Kenneth et al,
A.A. THORNTON & CO. Northumberland House 303/306
High Holborn
London, WC1V 7LE(GB)

(54) Drop dispenser.

(57) A dispenser device (A) for dispensing droplets of a liquid from a compressible container (B) having an open neck end (10) comprises a closure assembly (14) including a cap member (20) for liquid-tight attachment to the neck end (10) of the container and an elongated dispensing tube (56) projecting endwise from the cap member. The internal passageway (62) of the dispensing tube is packed with a multiplicity of solid cylindrical filaments (64) extending therethrough. The interstices between the filaments create a plurality of passageways (68) of minute cross-section extending through the tube for retarding the flow of liquid therethrough.

FIG.2

EP 0 093 493 A1

# DROP DISPENSER
## Background

The present invention pertains to the art of liquid dispensers, and more particularly to a dispensing device for accurately dispensing small droplets of liquid. The invention is particularly applicable for use as an eye dropper and will be described with particular reference thereto although it will be appreciated that the invention has other and broader applications.

## Background of the Invention

Medicant drop dispensers of the type to which this invention pertains are available in various sizes and shapes for the numerous medicines and solutions which are available for the care and comfort of the human eye. Heretofore, such dispensers have basically comprised a relatively small compressible plastic container or vial provided with a dispensing cap. The cap is generally provided with a dispensing nozzle having a small orifice or opening therein.

One of the special problems associated with these prior type drop dispensers is the awkwardness of their use. Because of the basic shape and design of these dispensers, the medicant was usually applied in either one of two different ways. With the first method, the medicant is applied by tilting the head backward and opening the eyelid. The nozzle of the dispenser is held a few inches away from and above the surface of the eye, and the drops are applied by squeezing the plastic container. The number of drops applied depends upon the size of the opening in the cap and the pressure exerted on the plastic container.

Another way in which eye drops from such prior dispensers have been dispensed is to insert the drops onto the inside of the lower eyelid. This method requires the head to be slightly tilted and the lower eyelid pulled away from the surface of the eye. The drops are then inserted on the lower lid. With this method, a mirror is generally required to locate the drop applicator with relation to the eye.

Though neither of these methods is especially complicated, they do create problems for certain individuals. People of advanced age, or those with severe vision impairment generally require corrective lenses (e.g., eyeglasses) to correct poor eyesight. For these elderly or visually handicapped persons, it is extremely difficult to focus on objects even a few inches away from the eye without such corrective eyeglasses, and the methods of applying medicine described above require the removal of such corrective eyeglasses. For these individuals, the methods of applying eyedrops as previously described are extremely difficult if not impossible to perform, and generally require the assistance of another person.

Another problem associated with conventional eye droppers is the difficulty of accurately controlling the amounts of medicine dispensed, i.e., the number and size of drops dispensed. Accurately controlling the rate and placement of the drops is very important. Most medicants for the human eye are extremely expensive; a single drop can cost upward of several dollars each. The loss or waste of even a few drops therefore can represent a substantial expense.

Most conventional eyedrop dispensers heretofor provide a minute orifice or opening in the nozzle portion of the cap. The larger the opening the less pressure required to be exerted on the container and the more freely the medicant is dispensed. To provide a slower dispensing rate, the orifice in the nozzle is usually restricted, which then requires more pressure to be exerted on the container to force the medicant through the smaller opening. This type of dispenser does not provide the accuracy and control necessary for today's expensive medicants.

0093493

-3-

In addition, for many persons such as the elderly, it is difficult to self-employ conventional eyedroppers, especially when dispensing the drops in the manner previously described. For these individuals who generally live on a fixed income, the loss or waste of a few drops can create a substantial financial burden.

## The Invention

The present invention contemplates a new and improved dispenser device for dispensing droplets of a liquid which overcomes all of the above-referred to difficulties and provides a liquid medicant dispenser which accurately dispenses droplets of liquid medicine at desired locations without waste of expensive medicant and without requiring removal of corrective eyeglasses, and which dispenser is simple, accurate, economical, and easy to use.

In accordance with the present invention there is provided, for use with a compressible container from which liquid droplets such as eye drops are to be dispensed, a dispensing device comprising a closure assembly including a cap member for liquid-tight attachment to an open neck end of the container and an elongated dispensing member extending endwise from the cap member and terminating in a tip end from which the liquid droplets are dispensed. The elongated dispensing member is in the form of a relatively long and somewhat flexible tube suitably formed of a plastic material and of sufficiently small cross-sectional size and sufficient length to enable the dispensing tip end to be positioned closely adjacent a person's eye while corrective eyeglasses remain in place. By employing such a small cross-section dispensing tube of extended length such that the dispensing tip end of the tube is located at some distance from the container holding the liquid to be dispensed, both the container and the hand of a person holding the container then can be positioned to the side of or above the eye into which the liquid droplets are to be dispensed so as not to interfere with a person's vision when utilizing a mirror to locate the dispensing tip end near the eye.

Further in accordance with the present invention, the elongated dispensing member contains a plurality of passageways of minute cross-section extending therethrough, which provide an increased ratio of surface area to flow cross-sectional area over that provided by a single passageway of equivalent flow cross-sectional area. This increased surface area strengthens the surface tension adhering forces exerted on the fluid medicant passing through the elongated dispensing member. By providing a plurality of minute passageways as opposed to a single large passageway, the additional surface area provides greater surface tension adhering force to control the dispensing of the liquid from the tip end of the dispensing member. In accordance with the preferred embodiment, these passages are defined by long, solid filaments of generally circular cross-section extending longitudinally through a generally flexible dispenser tube. The interstitial spaces defined by the surface of the filaments and the internal surfaces of the dispensing tube form the passageways therethrough.

## Objects

The principal object of the invention is the provision of a new and improved device for dispensing drops of liquid medicant which is simple and economical in construction and easy to use.

Another object of the invention is the provision of an eye drop dispenser which enables medicant drops to be inserted into the eye without the loss of any medicant and without requiring removal of corrective eyeglasses.

A further object of the invention is the provision of an improved drop dispenser which affords more accurate control over the amount of liquid medicant dispensed therefrom.

## Drawings

The invention may take physical form in certain parts and arrangements of parts a preferred embodiment of which will be described in detail in this specification and illustrated in the accompanying drawings wherein;

FIGURE 1 is a perspective view of a liquid medicant dispenser utilizing the preferred embodiment of the present invention;

FIGURE 2 is an enlarged cross-sectional view of the preferred embodiment;

FIGURE 3 is an enlarged cross-sectional view taken generally along the lines 3-3 of FIGURE 2; and,

FIGURE 4 is an enlarged longitudinal sectional view of the dispensing tip end portion of the elongated dispensing member shown in FIGURES 2 and 3.

## Preferred Embodiment

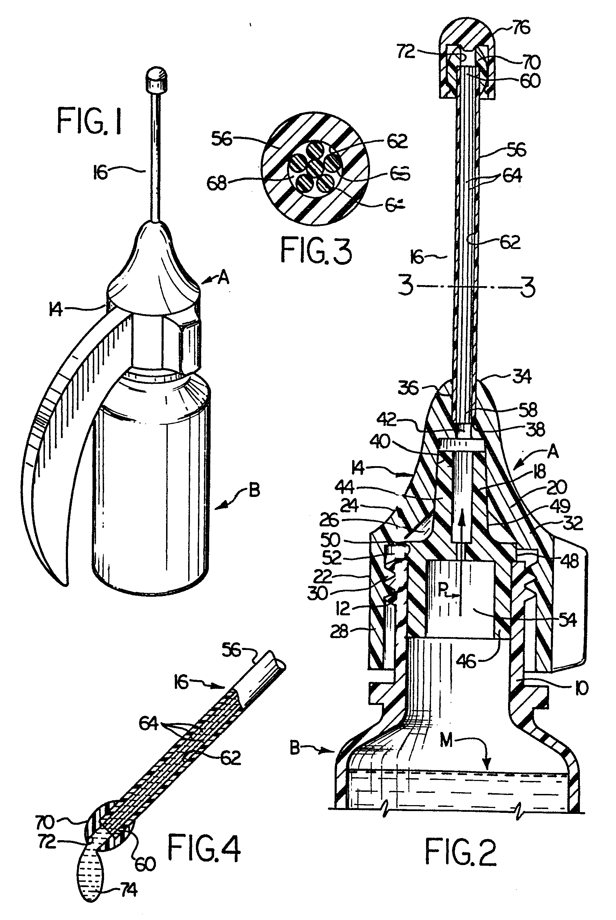

Referring now to the drawings wherein the showings are for the purpose of illustrating a preferred embodiment of the invention only and not for the purpose of limiting same, FIGURE 1 shows an assembly of a liquid drop dispensing device A comprising the invention with a compressible plastic container or vial B containing a supply of liquid medicant M to be dispensed. Container B forms no part of the present invention and is shown relatively conventionally. Normally, any plastic container or vial B such as commonly used in conjunction with an eye drop dispenser would include a reduced diameter open neck end portion 10 provided with external screw threads 12 over the uppermost part of the neck end portion. The screw threads 12 are adapted to matingly engage internal threads on dispensing device A to thereby attach the latter in place on the container B. Device A is comprised of a closure assembly 14 and an elongated dispensing member 16.

As shown in FIGURE 2, the closure assembly 14 is comprised of an insert 18 and a cap 20 both of which may be made of a suitable plastic material. A passage shown by arrow P in Figure 2 is provided axially through the closure assembly 14. Portions of passage P are formed in the insert 18 and the cap 20 and will be described in more detail subsequently. The cap 20 comprises a generally cylindrical mounting portion 22 and a nozzle end portion 24. A passageway 26 of varying diameter is provided axially through the cap. The cylindrical mounting portion 22 of the cap has an annular wall section 28 provided with internal helical screw threads 30 for mating engagement with the screw threads 12 on the neck end portion 10 of the container B to attach the cap thereto. The nozzle end portion 24 of the cap 20 is of generally conical shape having a base section 32 generally tapering to an outer apex end 34. The passageway 26 through the cap 20 decreases in diameter from the base 32 to the apex 34 of the conical section of the cap, as seen in FIGURE 2. From the apex 34 of the nozzle 24 the passageway 26 thereof is counterbored inwardly to provide a recess 36 and radial shoulder 38.

Midway through the conical nozzle end section 24 of the cap 20 the internal passageway 26 thereof is formed with a frusto-conical shaped section 40 which wedgingly engages with the insert 18. The cap portion of path P is defined by the passageway 42 through the apex end portion 34 of the nozzle end 24.

Insert 18 has a cap contacting portion 44, a container neck contacting or stopper portion 46, and an intermediate annular collar portion 48. The cap contacting portion 44 is formed of frusto-conical exterior shape as shown at 49 to wedgingly mate with the frusto-conical internal surface 40 of the conical portion 24 of the cap 20. The container contacting portion 46 of the insert 18 has an external diameter slightly less than the inner diameter of the open neck end portion 10 of the container B, which allows a close sliding fit of the insert 18 into the open neck end 10 of the container. The annular collar 48 is located between and joins the cap contacting portion 44 and the container contacting portion 46 of the insert 18. The collar 48 has a flat annular abutment shoulder 50 which engages the end face or rim 52 of the container neck 10. When the cap 20 is threaded on the container B, the wedging action between the surfaces 40 and 49 of the cap 20 and the insert 18 forces abutment shoulder 50 and end face 52 together to provide a liquid-tight seal therebetween. A passageway 54 of varying diameter is formed axially through the insert 18. The passageway 54 forms the portion of passageway P within the insert 18.

In axial alignment with the path P is elongated dispensing member 16. In the preferred embodiment, the elongated dispensing member is comprised of a hollow, somewhat flexible tube 56 of a suitable plastic material at least two inches in length. The tube 56 has an entrant end 58 and a dispensing end 60 and an inner passageway or bore 62 extending therethrough. The entrant end 58 of the dispensing tube 56 is force fitted into recess portion 36 of the passageway 42 in the cap 20, and in abutting engagement with the radial shoulder 38 in the recess 36, to firmly secure the dispensing tube in place on the cap 20 in endwise extending and fluid-tight relation thereto. As best shown in FIGURE 3, the dispensing tube 56 is packed or filled with a plurality of fine plastic filaments or fibers 64 of generally circular cross-section having external surfaces 66 and extending longitudinally throughout the entire length of the passageway 62 of the dispensing tube. The interstitial spaces formed between the adjoining circular or otherwise rounded contour surfaces 66 of the filaments 64 themselves, and between the filaments and the wall of the passageway 62 of the tube 56, define a plurality of extended passageways 68. These passageways 68, as best seen in FIGURE 3 have minute cross-sectional areas which are of a magnitude of less than .001 square inches. The cross-sections have irregular shapes which expose additional solid surface area to the liquid and create a capillary effect within each passageway. A rounded, smooth surfaced protective tip 70 of a suitable soft plastic material, for example, is fixedly secured to the dispensing tip end 60 of the tube 56 in fluid-tight relation thereto. The protective tip 70 is provided with a dispensing orifice 72 aligned with the passageway or bore 62 of the dispensing tube 56, from which orifice 72 the droplets 74 of liquid medicant are expelled when the container B is manually compressed or squeezed. A cover cap 76 suitably made of a plastic material is provided to fit tightly over the protective tip 70 and seal off the orifice 72 thereof when the dispensing device is not in use.

With the present invention, drops of medicant can be inserted into the eye easily and accurately without the loss of expensive medicant such as commonly employed for eyedrops. For individuals with poor eyesight, who require the aid of corrective eyeglasses for at least minimal vision, the elongated dispensing member 16 of the present invention enables the dispensing end 60 to be inserted between the eyeglasses and the surface of the eye. Only a portion of tube 56 and the protective tip 70 is therefore positioned in front of the eye. The hand of the person holding the container B and the assembly A, B in droplet dispensing position is thus located off to the side of or above the eye and will not impair the vision of a person looking into a mirror to locate the protective tip 70 relative to the eye. In addition, the frames of any corrective eyeglasses being worn can be used as a pivot point to steady the elongated dispensing tube 16 and maintain it in proper droplet dispensing position relative to the eye.

With regard to the filaments or fibers 64 contained within elongated dispensing member 16, the additional surface area 66 created within the tube 56 by the filaments 64 increases the surface tension adhering force exerted on the liquid as it is forced through the tube, and therefore provides more accurate control over the amount of liquid medicant dispensed. It is appreciated that other embodiments of the present invention exist and that ther additional modifications and alterations will occur to others upon their reading and understanding of the specification. It is intended that all such modifications and alterations be included insofar as they come within the scope of the appended claims or the equivalents thereof.

**0093493**

CLAIMS:

1. A dispenser closure for a compressible container for accurately dispensing small droplets of liquid medicant therefrom , said closure comprising a cap member having a passage therethrough and provided with means for securing said closure liquid-tight to said container with said passageway communicating with the interior of said container, and an elongated hollow dispenser member secured liquid-tight to and extending from said cap member in alignment with said passage, said dispensing member having a plurality of passageways of minute cross-sectional area extending therethrough.

2. The closure defined in claim 1 wherein said elongated dispensing member is a generally flexible hollow tube containing a plurality of stationary fluid obstructions wherein the space between said obstructions define said passageways.

3. The closure defined in claim 2 wherein said obstructions are long solid filaments of generally round cross-sectional shape extending longitudinally through said hollow tube.

4. The closure defined in claim 3 wherein said container is bottle-shaped having an extending threaded neck portion.

5. The closure defined in claim 4 wherein said elongated dispensing member is at least two inches in length.

6. The closure defined in claim 5 wherein said elongated dispensing member is provided with a smooth rounded generally soft member at the dispensing end of said dispensing member.

7. A liquid medicant dispensing closure for use with a compressible plastic container, said closure comprising a cap member having a passage therethrough and means for securing said closure liquid tight to said container, and an elongated generally flexible tubular member secured liquid tight to and extending from said cap member in alignment with said passage, said tubular member containing material within the passageway thereof providing additional solid surface area within said tubular member over that provided by the inner wall of said tubular member.

8. The closure defined in claim 7 wherein said filaments extend longitudinally through said hollow tube.

0093493

1 / 1

FIG.1

FIG.3

FIG.4

FIG.2

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y,A | US-A-2 605 937 (BRADLEY) * Claim 1; figure 2 * | 1-3,7 8 | A 61 F 9/00 A 61 J 1/00 B 65 D 47/18 |
| Y,A | FR-A-1 168 219 (MARTIN et al.) * Whole document * | 1,7 | |
| Y,A | DE-A- 19 380 (ATLAN-WERK L. SATTLER KG) * Claim 1 * | 1,7 | |
| A | US-A-2 874 881 (STULL) | | |
| A | FR-A- 968 572 (FABERGE) | | |
| A | FR-A-1 333 865 (ANCIENS ETS E. ROBERT, F. VAUTHIER & CIE SUCCESSEURS) | | **TECHNICAL FIELDS SEARCHED (Int. Cl. ³)** |
| A | DE-C-1 063 755 (ATLAN-WERK L. SATTLER KG) | | A 61 F 9/00 A 61 J 1/00 B 65 D 47/00 |

The present search report has been drawn up for all claims

| Place of search BERLIN | Date of completion of the search 18-07-1983 | Examiner KANAL P K |
|---|---|---|